# FASCICULE DE BREVET EUROPEEN

(11) **EP 1 299 081 B1**
(45) Date de publication et mention de la délivrance du brevet: **16.01.2008**
(21) Numéro de dépôt: 01953195.3
(22) Date de dépôt: 05.07.2001
(51) Int. Cl.: A61Q 19/00, A61K 8/60

(54) **UTILISATION D'OLIGOSACCHARIDES POUR STIMULER LA PRODUCTION DE BETA-ENDORPHINE**
VERWENDUNG VON OLIGOSACCHARIDEN ZUR STIMULIERUNG DER PRODUKTION VON BETA-ENDORPHIN
USE OF OLIGOSACCHARIDES TO STIMULATE BETA-ENDORPHIN PRODUCTION

(30) Priorité: 07.07.2000 FR 0008879
(43) Date de publication de la demande: 09.04.2003
(73) Titulaire: LVMH RECHERCHE, 45800 St. Jean de Braye (FR)
(72) Inventeur: BONTE, Frédéric, 45100 Orleans (FR); DUMAS, Marc, 45100 Orleans (FR); LHERMITE, Stéphane, 45400 Semoy (FR); SAUNOIS, Alex, 45100 Orleans (FR)
(74) Mandataire: Portal, Gérard
(86) Numéro de dépôt international: PCT/FR2001/002154
(87) Numéro de publication internationale: WO 2002/003945

(56) Documents cités:
- EP-A- 0 610 511
- WO-A-95/05155
- FR-A- 2 708 198
- FR-A- 2 762 783

## Description

La présente invention concerne essentiellement l'utilisation d'oligosaccharides contenant au moins deux motifs galactose tels que spécifiés ci-après ou un extrait de plantes en contenant, comme agent cosmétique

La présente invention concerne essentiellement l'utilisation d'oligosaccharides contenant au moins deux motifs galactose tels que spécifiés ci-après ou un extrait de plantes en contenant, comme agent cosmétique ainsi qu'une méthode de soins cosmétiques en comportant application. Plus particulièrement, l'invention concerne l'utilisation d'oligosaccharides comprenant de 2 à 6 sucres et contenant au moins deux motifs galactose, de préférence deux motifs galactose vicinaux et de préférence encore deux motifs galactose vicinaux en bout de chaîne, ou d'un extrait de plantes en contenant, comme agent cosmétique, notamment pour stimuler la production de bêta-endorphine dans la peau, de préférence pour stimuler la production de bêta-endorphine par les kératinocytes dans la peau, ainsi qu'une méthode de soins cosmétiques, en comportant l'application.

Les oligosaccharides concernés font le D-stachyose, plus communément appelé stachyose, ou encore appelé 0-α-D-galactopyranosyl-[1→6]-0-α-D-galactopyranosyl-[1→6]-β-D-fructofuranosyl-α-D-glucopyranoside, de formule chimique condensée C₂₄ H₄₂ O₂₁, disponible dans le commerce, et le cicéritol ou 0-α-D-galactopyranosyl-[1→6]-0-α-D-galactopyranosyl-[1→2]-4-0-méthyl-D-chiro-inositol, de formule chimique condensée C₁₉ H₃₄ O₁₆.

Ces oligosaccharides peuvent être isolés à partir de plantes, en particulier d'une plante du genre Tephrosia, en particulier de l'espèce Tephrosia purpurea.

Ces oligosaccharides peuvent également être isolés d'une plante du genre soja, pois chiche, lupin ou de lentilles.

L'utilisation d'extraits de Tephrosia, en particulier de Tephrosia purpurea a déjà été décrite dans le document FR-2 708 198 B pour la préparation d'une composition cosmétique ou pharmaceutique, notamment dermatologique et un procédé de traitement cosmétique en comportant application sur la base de la découverte inattendue qu'un tel extrait présentait une activité de stimulation puissante de l'enzyme adénylate cyclase.

Dans le cadre de la présente invention, il a maintenant été découvert de manière totalement inattendue que ces oligosaccharides comprenant de 2 à 6 sucres et ayant au moins 2 motifs galactose, de préférence 2 motifs galactose vicinaux et de préférence encore deux galactoses vicinaux en bout de chaîne, ou un extrait de plantes en contenant, et notamment un extrait de la plante Tephrosia, en particulier Tephrosia purpurea, étaient capables de stimuler la production de β-endorphine dans la peau, de préférence en étant capables de stimuler la production de β-endorphine par les kératinocytes dé la peau.

Ainsi, la présente invention a pour but principal de résoudre le nouveau problème technique consistant en la fourniture d'une solution qui permette de fournir de nouveaux agents cosmétiques de nouvelles compositions cosmétiques, capables de stimuler la production de β-endorphine dans la peau, notamment de stimuler la production de β-endorphine par les kératinocytes de la peau.

La présente invention a encore pour but principal de résoudre le nouveau problème technique consistant en la fourniture d'une solution qui permette de fournir de nouveaux agents cosmétiques capables de soigner les peaux sensibles, de combattre la sensibilité cutanée.

Ces problèmes techniques sont résolus pour la première fois par la présente invention de manière particulièrement simple, fiable et reproductible, utilisable à l'échelle industrielle.

Ainsi, selon un premier aspect, la présente invention couvre l'utilisation telle que définie dans la revendication 1. Selon un deuxième aspect, l'invention couvre un procédé non thérapeutique de soin cosmétique tel que définie dans la revendication 12.

Selon un autre mode de réalisation avantageux, l'utilisation est caractérisée en ce que l'oligosaccharide précité est le stachyose.

Selon un autre mode de réalisation avantageux de l'invention, l'oligosaccharide précité est le cicéritol.

Dans le cadre de l'invention, selon un mode de réalisation avantageux, on peut utiliser un extrait de graines de plante contenant l'oligosaccharide défini précédemment, de préférence la plante étant choisie parmi le groupe constitué par la plante Tephrosia, le soja, le pois chiche, le lupin, et la lentille. De préférence encore, l'extrait de plante est extrait de graines de l'espèce Tephrosia purpurea.

Selon un mode de réalisation avantageux, l'extrait de graines est un extrait hydro-alcoolique avec un alcool en C₁-C₆ linéaire ou ramifié ou cyclique. Un alcool particulièrement préféré est le méthanol, l'éthanol ou le butanol. Les proportions relatives eau-alcool peuvent varier dans de larges limites. Le mélange actuellement préféré est environ 2/3 d'alcool pour 1/3 d'eau, proportion relative en poids. Pour cette extraction, on peut utiliser un rapport masse de solvant/masse de matières premières de 5/1 environ à 50/1 environ et plus, et de préférence de l'ordre d'environ 12/1.

L'extraction peut être réalisée à température ambiante ou avec tout chauffage modéré, en particulier à une température d'extraction comprise entre 20°C et 70°C et de préférence à environ 45°C. On obtient ainsi un produit concentré dans l'eau et il est possible de le ressolubiliser par exemple après addition du même alcool ou d'un alcool différent, notamment du propylène glycol ou d'éthanol, ou d'eau en grand volume. Il est actuellement avantageux d'effectuer une triple formulation du produit dans un mélange alcool/eau de l'ordre de 30:70 en poids, l'alcool étant de préférence le propylène glycol. On peut aussi ajouter un tensioactif tel que le Phénonip^{®}.

L'extrait obtenu est composé essentiellement d'oligosaccharides et plus précisément de fructose, de saccharose, de raffinose, de stachyose et de cicéritol.

Selon encore un autre mode de réalisation avantageux de l'invention, l'oligosaccharide précité ou un extrait de plantes en contenant est combiné à une autre substance active cosmétiquement ou dermatologiquement acceptable, de préférence choisie parmi le groupe constitué de la vitamine A et ses esters, en particulier le palmitate de la vitamine A, un acide alphahydroxylé en particulier l'acide salicylique et ses dérivés, l'acide lactique, glycolique, malique ; une substance inhibitrice de l'enzyme PLA2, telle qu'un extrait de la plante Phellodendron amurense, de la plante Azadirachta indica ; d'une substance à activité anti-inflammatoire telle que l'acide 18 β-glycyrrhétinique, un extrait de la plante glycyrrhizaglabra, une substance à activité immunomodulatrice telle qu'un glycane ; un agent tensioactif en particulier de la famille du laurylsulfate ; une substance alcaloïde de préférence une bisbenzyl isoquinoléine, en particulier oxyacanthine, cépharanthine ; une substance inhibitrice de PAF, en particulier un extrait de Gingko biloba, une substance inhibitrice d'enzymes PGE2.

Selon encore un autre mode de réalisation avantageux de l'invention, l'oligosaccharide précité, ou un extrait de plantes en contenant, est appliqué sur la peau pour réaliser le soin de peaux sensibles, notamment pour réduire ou supprimer les réactions d'inconfort, pour procurer des sensations de bien-être.

Dans l'un quelconque des aspects précédents, selon un autre mode de réalisation avantageux, on utilisera de 0,0001 % à 10 %, de préférence 0,01 à 5 % en poids d'oligosaccharides ou d'extraits en contenant, exprimés en poids sec, par rapport au poids total de la composition.

D'autres buts, avantages de l'invention apparaîtront clairement à la lumière de la description explicative qui va suivre faite en référence à divers exemples de réalisations de l'invention donnés simplement à titre d'illustration. Cependant, les exemples font partie intégrante de la présente invention et Dans la description et les revendications et sauf indications contraires, les pourcentages sont donnés en poids, les températures sont en degrés celsius, et la pression est la pression atmosphérique.

### EXEMPLE 1

### Fabrication d'un extrait riche en oligosaccharides, en particulier en oligosaccharides ayant 2 motifs galactose vicinaux situés en bout de chaîne (produit I_{L}de l'invention).

On prend 100 grammes de graines de Tephrosia purpurea achetées dans le commerce que l'on broie et tamise à 1 mm. On introduit ces graines broyées dans un mélange de solvant d'extraction et d'eau comprenant environ 0,84 kg d'alcool et environ 0,36 kg d'eau distillée pour fournir un mélange d'extraction hydro-alcoolique à environ 70 % en poids.

Dans le cadre de cet exemple, l'alcool d'extraction est l'éthanol, à 96 % v/v.

On réalise une extraction avec ce mélange d'extraction pendant environ 5 heures à environ 45°C sous agitation.

Ensuite, on refroidit le mélange jusqu'à la température ambiante, soit à environ 25°C. On filtre sous vide avec un filtre ayant des pores à environ 11 µm.

On réalise dans le même réacteur une concentration sous vide, sous une pression comprise entre 160 et 60 mbars et avec une température du bain de concentration sous vide d'environ 58°C, jusqu'à obtenir un concentré de 80 g environ.

Après évaporation, on effectue une remise en solution, toujours dans le même réacteur, par ajout d'un alcool, ici du propylène glycol de qualité cosmétique, à une quantité d'environ 30 g, sous une vive agitation pendant environ 20 mn.

Ce produit peut être utilisé tel quel ou bien être purifié en procédant à un ajout dans la solution de 14 g de charbon actif du commerce (référence C x V, Société CECA, France) et que l'on soumet à une agitation pendant 15 mn à température ambiante.

On réalise ensuite une filtration classique sous vide sur un filtre ayant un diamètre de pores de 5 µm.

Le poids de la solution filtrée contenant les oligosaccharides est d'environ 70 g. La filtration a lieu sans problème. On ajuste la teneur en matière sèche à environ 5 % par addition d'un mélange propylène glycol/eau 30:70 en poids.

On procède à une nouvelle filtration sur un filtre ayant un diamètre de pores de 0,22 µm puis on peut éventuellement et avantageusement ajouter un tensioactif tel que Phénonip^{®} à raison de 0,5 % en masse. Le produit ainsi obtenu est dénommé produit I₁ de l'invention.

### EXEMPLE 2

On procède comme décrit l'exemple 1, si ce n'est que l'on utilise du butanol comme alcool d'extraction.

On obtient un produit de l'invention dénommé produit I₂.

### EXEMPLE 3

On procède comme décrit l'exemple 1, si ce n'est que l'on utilise comme alcool d'extraction du méthanol.

On obtient un produit selon l'invention dénommé produit I₃.

### EXEMPLE 4

### Préparation de cicéritol purifié à partir de graines de Tephrosia purpurea

On procède comme décrit à l'exemple 1 jusqu'à l'étape de concentration sous vide jusqu'à environ 80 g de concentré dans le réacteur, la température du bain de concentration sous vide étant d'environ 58°C et la pression du vide étant comprise entre 160 et 60 mbars.

On ajoute alors 14 g de charbon actif du commerce (référence C x V de la Société CECA, France), et l'on soumet à une agitation pendant 15 min à température ambiante.

On réalise ensuite une filtration sous vide de manière classique sur un filtre ayant un diamètre de pores de 5 µm.

On réalise ensuite une nouvelle filtration sur un filtre ayant un diamètre de pores de 0,2 µm puis on effectue une évaporation à sec.

Le rendement obtenu par rapport au sec est de 7 % en poids.

On procède ensuite à une chromatographie liquide préparative haute performance, de la manière suivante :

### Conditions expérimentales

On utilise une colonne en acier à compression axiale (D.I. = 4 cm/ longueur = 30 cm)
Phase stationnaire : Lichrospher 100 DIOL^{®} 15 µm (Merck)
Conditionnement de la colonne : 200 g de phase stationnaire sont dispersés dans de l'acétonitrile
Pression/compression : 100 bars
Quantité injectée : 630 mg d'extrait séché dilués dans 3 ml d'eau
Gradient d'élution : acétonitrile 95 acétonitrile : 50 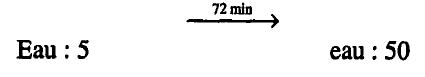
Débit d'élution : 100 ml.min⁻¹
Détection : détecteur à diffusion de lumière photomultiplicateur SEDEX 55 (Société Sedere, Alfortville, France) (PM 4 à 2,5 bars d'air et 45°C).

Le produit purifié élué est évaporé puis lyophilisé de manière à obtenir une poudre de couleur blanche ayant un point de décomposition de 160°C, un pouvoir rotatoire [α]²⁵*_{D}* de + 159,01° dans l'eau à 0,93 g/ml.

Le rendement de purification est d'environ 7 %, ce qui donne un rendement global par rapport à l'extrait sec de 0,49 %, la pureté étant supérieure à 90 %. Le contrôle de pureté est réalisé de manière similaire par chromatographie liquide haute performance sur la même colonne analytique de type DIOL^{®}, ce contrôle montrant la présence d'une seule molécule avec une pureté très élevée. Une étude structurale, par RMN d'une part et par spectrographie de masse d'autre part, a permis de confirmer que le composé obtenu avait bien la structure du cicéritol.

Ce produit ainsi obtenu est utilisé dans les essais de l'exemple 5 suivant.

### EXEMPLE 5

### Mise en évidence de l'action stimulante des extraits de Tephrosia purpurea, du stachyose et du cicéritol pour la synthèse de bêta-endorphine par des kératinocytes humains normaux

Il est décrit dans la littérature notamment dans J. Invest. Dermatol. 1996, 106, 673-678 ; J. Clin. Invest.1994, 93, p.2258-2262 que les kératinocytes humains, cellules qui constituent l'un des composants essentiels de l'épiderme, sont capables de synthétiser et sécréter certaines neurohormones telles que la bêta-endorphine. La bêta-endorphine est un dérivé de la propiomélanocortine (POMC) qui a très probablement un rôle dans les phénomènes d'immunomodulation et dans le cycle pilaire comme décrit dans la littérature notamment dans J. Invest. Dermatol. 1996, 106, 3-10 ; Biochim. Biophys. Acta 1997, 1336, p.315-322.

Il a été émis l'hypothèse que la libération instituée par les kératinocytes de bêta-endorphine était suffisante pour passer dans le sérum et agir à longue distance sur le système nerveux central et les cellules immunes circulantes comme décrit dans J. Invest. Dermatol 1996, 106, 673-678.

La POMC, hormone originalement découverte au niveau de la glande pituitaire exerce les fonctions de précurseurs de neuropeptides. Les neurohormones sont libérées dans l'organisme au cours de situations de stress, d'irradiation UV et ont des effets antalgiques qui peuvent être importants pour le développement de produits cosmétiques destinés en particulier aux peaux sensibles.

Il a été démontré dans la littérature un rôle spécifique de la β-endorphine par rapport aux enképhalines. Par exemple, Nissen J B et al. ont démontré dans Exp. Dermatol. 1997, 6, 222-229 que contrairement aux enképhalines la β-endorphine ne possédait pas de rôle sur la différenciation des kératinocytes.

Dans le présent essai, les inventeurs ont mis en évidence de manière totalement inattendue que des oligosaccharides ayant au moins 2 sucres galactose vicinaux de préférence en bout de chaîne, en particulier les oligosaccharides présents dans les extraits de Tephrosia purpurea, en particulier le stachyose et le cicéritol, avaient la capacité de stimuler de manière significative la synthèse de β-endorphine par des kératinocytes humains normaux. Le test d'activité est le suivant :

### TEST D'ACTIVITE

### Produits testés

On utilise soit des extraits hydro-alcooliques de Tephrosia purpurea obtenus selon le procédé de l'exemple 1, soit du stachyose disponible dans le commerce (chez Sigma, France), soit du cicéritol, isolé à partir de l'extrait hydro-alcoolique I₁ de tephrosia purpurea de l'exemple 1, comme décrit à l'exemple 4.

### Test cellulaire

Des kératinocytes humains normaux sont cultivés jusqu'à confluence sur une plaque de 24 puits puis incubés dans un milieu de culture pendant 24 heures en présence de dibutyryl AMPc (2mM), d'interleukine 1-β (IL- 1-β) (500 pg/ml), et du produit à tester, ici à savoir soit des extraits de Tephrosia purpurea selon l'exemple 1, soit le stachyose, soit le cicéritol, respectivement à la dose indiquée aux tableaux I et II.

### 5.1. ESSAI AVEC LES EXTRAITS DE TEPHROSIA

Chaque extrait de tephrosia obtenu selon le procédé de l'exemple 1 est fourni pesé exactement et redissous à la concentration de 50 mg/ml dans un mélange d'éthanol/eau (1:1).

On a procédé à la préparation de plusieurs lots d'extraction de graines de Tephrosia purpurea suivant la procédure d'extraction décrite à l'exemple 1, respectivement dénommées lots L1, L2 et L3. Pour le lot L3, on a procédé à deux séries d'essais.

Les résultats obtenus comme indiqué ci-après sont répertoriés au tableau I.

On ajoute des antiprotéases, aprotinine 5 µg/ml, leupeptine 1 µg/ml, PMSF 1 mM, dans chaque puits afin de limiter l'action des protéases.

Chaque point expérimental est réalisé en double.

Les surnageants de culture sont alors récupérés après 24 h d'incubation et congelés à - 80°C.

Des témoins positifs de stimulation sont réalisés en parallèle en traitant comme précédemment les cellules pendant 24 h avec soit l'IL-1-β, à la dose de 500 pg/ml, soit le dibutyryl AMPc 2 mM.

La β-endorphine secrétée est dosée par un dosage EIA, (kit de Peninsula Laboratories) et exprimée en pg/ml.

Ces résultats obtenus sont indiqués au Tableau I ci-après :

**TABLEAU I**

| **Conditions expérimentales de traitement des kératinocytes** | **β-endorphine sécrétée en pg/ml** | **Test t de Student valeur de p** |
|---|---|---|
| **Témoin éthanol à 0,025 %** | 36 ± 6 | |
| Tephrosia purpurea 1 µg/ml (L1) | 74 ± 8 | 0,0359 |
| Tephrosia purpurea 5 µg/ml (L1) | 98 ± 15 | 0,0323 |
| Tephrosia purpurea 25 µg/ml (L1) | 77 ± 11 | 0,0456 |
| **Témoin** | 3+4 | |
| Témoin positif : IL-1-β 500 pg/ml | 35 ± 5 | 0,0208 |
| Témoin positif : dibutyryl-AMPc 2 mM | 87 ± 12 | 0,0115 |
| | | |
| **Témoin** | 3 ± 4 | |
| Tephrosia purpurea 1 µg/ml (L2) | 26 ± 6 | 0,0442 |
| Tephrosia purpurea 5 µg/ml (L2) | 44 ± 7 | 0,0196 |
| Tephrosia purpurea 25 µg/ml (L2) | 26 ± 4 | 0,0324 |
| Témoin positif : IL-1-β 500 pg/ml | 35 ± 5 | 0,0208 |
| Témoin positif : dibutyryl-AMPc 2 mM | 87 ± 12 | 0,0115 |
| **Témoin** | 1 ± 1 | |
| Tephrosia purpurea 1 µg/ml (L3) | 3 ± 1 | 0,3081 |
| Tephrosia purpurea 5 µg/ml (L3) | 14 + 2 | 0,0047 |
| Tephrosia purpurea 25 µg/ml (L3) | 14 ± 2 | 0,0047 |
| Témoin positif : IL-1-β 500 pg/ml | 17 ± 3 | 0,0036 |
| Témoin positif : dibutyryl AMPc 2 mM | 10 ± 1 | 0,0065 |
| | | |
| **Témoin** | 1 ± 3 | |
| Tephrosia purpurea 1 µg/ml (L3) | 21 ± 5 | 0,0280 |
| Tephrosia purpurea 5 µg/ml (L3) | 21 ± 4 | 0,0179 |
| Tephrosia purpurea 25 µg/ml (L3) | 20 ± 1 | 0,0121 |
| Témoin positif : IL-1-β 500 pg/ml | 25 ± 4 | 0,0065 |
| Témoin positif : dibutyryl AMPc 2 mM | 22 + 1 | 0,0067 |

### 5.2 ESSAIS AVEC LE STACHYOSE ET LE CICERITOL

En procédant de la même manière que dans le cadre des essais avec les extraits de Tephrosia purpurea, mais en utilisant le stachyose et le cicéritol aux doses indiquées au tableau II, on obtient les résultats indiqués au tableau II ci-après.

**TABLEAU II**

| **Conditions expérimentales de traitement des kératinocytes** | **β-endorphine sécrétée en pg/ml** | **Test t de Student valeur de p** |
|---|---|---|
| **Témoin** | 10 ± 3 | |
| Stachyose 20 ng/ml (Sigma ref. S4001) | 16 ± 1 | 0,0733 |
| Stachyose 100 ng/ml (Sigma ref. S4001) | 22 ± 2 | 0,0086 |
| Stachyose 500 ng/ml (Sigma ref. S4001) | 30 ± 13 | 0,0289 |
| Témoin positif : IL-1-β 500 pg/ml | 25 ± 4 | 0,0065 |
| Témoin positif : dibutyryl-AMPc 2 mM | 22 ± 1 | 0,0067 |
| **Témoin** | 1 ± 2 | |
| Cicéritol 20 ng/ml | 8 ± 3 | 0,0380 |
| Cicéritol 100 ng/ml | 8 ± 4 | 0,0765 |
| Cicéritol 500 ng/ml | 9 ± 5 | 0,0863 |
| Témoin positif : IL-1-β 500 pg/ml | 17 ± 3 | 0,0036 |
| Témoin positif : dibutyryl-AMPc 2 mM | 10 ± 1 | 0,0065 |
| | | |
| **Témoin** | 3 ± 3 | |
| Cicéritol 64 ng/ml | 44 ± 7 | 0,0168 |
| Témoin positif : IL-1-β 500 pg/ml | 35 ± 3 | 0,0077 |
| Témoin positif : dibutyryl-AMPc 2 mM | 87 ± 8 | 0,0056 |

On notera que pour le cicéritol, on a réalisé deux séries d'essais pour lesquels il a également été effectué un témoin blanc et deux témoins positifs comme indiqué au tableau II, le cicéritol étant celui de l'exemple 4.

A partir des trois lots d'oligosaccharides de Tephrosia purpurea, objet des essais du tableau I, on observe une stimulation dose-dépendante de la libération de β-endorphine par les kératinocytes humains normaux dans la gamme 1-25 µg/ml.

L'effet maximal est obtenu à la concentration de 5 µg/ml.

Par ailleurs, il résulte des expériences réalisées à ce jour sur les deux molécules présentes dans les extraits de Tephrosia purpurea à savoir le stachyose et le cicéritol, objet du tableau II, qu'il a pu être démontré un effet de stimulation de la production de β-endorphine par ces mêmes cellules.

Dans les expériences rapportées dans le cadre de cet exemple, les témoins positifs IL-1-β et dibutyryl-AMPc ont également bien stimulé, mais avec des amplitudes différentes, en fonction des souches cellulaires utilisées, la production de β-endorphine des kératinocytes humains normaux en culture.

Dans ces conditions, les oligosaccharides, en particulier sous forme d'un extrait riche en oligosaccharides ici, un extrait de tephrosia, ou les substances isolées le stachyose et le cicéritol, induisent de manière significative la synthèse de β-endorphine, et peuvent être ainsi utilisés pour la fabrication de produits cosmétiques pour le soin des peaux sensibles, ces produits étant destinés à combattre la sensibilité cutanée et les réactions d'inconfort, pour procurer des sensations de bien-être, ou pour réaliser un effet apaisant

### EXEMPLE 6

### Lotion calmante après soleil

Cette lotion calmante est obtenue à partir des composants suivants, de manière classique bien connue de l'homme de l'art :
- extrait I₁ de Tephrosia purpurea de l'exemple 1 : 0,2
- céramide II : 0,5
- glycérine : 4
- acétate de tocophérol : 0,2
- extrait de réglisse : 0,2
- excipient : qsp 100

Cette lotion apaisante, utilisée après les bains de soleil calme la peau.

### EXEMPLE 7

### Gel raffermissant et relaxant pour le corps

Ce gel est préparé à partir des composants suivants :
- extrait I₁ de Tephrosia purpurea
   de l'exemple 1 : 1
- madécassoside : 0,2
- extrait de Sapindus mukurossi : 0,2
- protéines de blé : 2
- glycérine : 2
- excipient gélifiant : qsp 100

Ce gel raffermissant pour le corps libère au cours de l'application par massage des sensations de bien être et de plaisir.

### EXEMPLE 8

### Emulsion pour la nuit à effets tenseur et relaxant

Cette émulsion est préparée à partir des composants suivants :
- cicéritol de l'exemple 4 : 0,1
- madécassoside : 0,1
- sapindosides : 0,1
- OPC de pépin de raisin : 0,5
- excipient émulsionné : qsp 100
   Cette émulsion fine aux effets tenseurs est appliquée sur l'ovale du visage, repose les traits, améliore le bien être cutané et redonne un visage plus jeune au matin.

### EXEMPLE 9

### Crème de massage tonifiante et pour peaux sensibles et irritables

- extrait I₁ de Tephrosia purpurea de l'exemple 1 : 1
- extrait de ginseng : 0,1
- ergothionéine : 0,2
- excipient gras émulsionné pour massage : qsp 100

Cette crème pour peaux sensibles tonifie l'épiderme et relaxe le corps.

### EXEMPLE 10

### Emulsion fine régénérante et relaxante pour peaux sensibles et fragiles

- stachyose : 0,35
- palmitate de rétinol : 0,1
- acétate de tocophérol : 0,2
- soja sapogénols : 0,1
- madécassoside : 0,1
- filtres solaires : 8
- excipient : qsp 100

Cette émulsion fine régénérante stimule le métabolisme épidermique et redonne éclat et jeunesse.

## Revendications

1. Utilisation non-thérapeutique dans une composition cosmétique d'au moins un agent actif choisi dans le groupe du stachyose, du cicéritol et des extraits de plantes contenant du stachyose ou du ciceritol, en tant qu'agent cosmétique pour le soin des peaux sensibles ou pour combattre la sensibilité cutanée, par stimulation de la production de bêta-endorphine dans la peau.

2. Utilisation selon la revendication 1, **caractérisée en ce que** ledit agent actif agit sur la production de la bêta-endorphine par les kératinocytes de la peau.

3. Utilisation selon l'une des revendications 1 ou 2, **caractérisée en ce que** ledit agent actif est utilisé en tant qu'agent de soin des peaux sensibles.

4. Utilisation selon l'une des revendications 1 à 3, **caractérisée en ce que** ledit agent actif est le stachyose.

5. Utilisation selon l'une des revendications 1 à 3, **caractérisée en ce que** ledit agent actif est le ciceritol.

6. Utilisation selon l'une des revendications 1 à 3, **caractérisée en ce que** ledit agent actif est contenu dans un extrait de plantes choisies dans le groupe constitué de la plante Tephrosia, du soja, du pois chiche, du lupin et de la lentille.

7. Utilisation selon la revendication 6, **caractérisée en ce que** ledit extrait de plantes est un extrait de Tephrosia purpurea.

8. Utilisation selon l'une des revendications 6 ou 7, **caractérisée en ce que** ledit extrait est un extrait de graines de Tephrosia purpurea.

9. Utilisation selon l'une des revendications 1 à 8, **caractérisée en ce que** ledit agent actif est contenu dans une composition cosmétique, à une concentration exprimée en poids sec comprise entre 0,0001 % et 10 % par rapport au poids total de ladite composition.

10. Utilisation selon la revendication 9, **caractérisée en ce que** ladite concentration est comprise entre 0,01 % et 5 % en poids par rapport au poids total de ladite composition.

11. Utilisation selon l'une des revendications 1 à 10, **caractérisée en ce que** ledit agent actif est combiné à une autre substance active cosmétiquement acceptable, de préférence choisie dans le groupe constitué d'un acide alphahydroxylé en particulier l'acide salicylique et ses dérivés, l'acide lactique, glycolique, malique ; une substance inhibitrice de l'enzyme PLA2, telle qu'un extrait de la plante Phéllodendron amurense, de la plante Azadirachta indica ; un agent tensioactif en particulier de la famille du laurylsulfate ; une substance alcaloïde de préférence une bisbenzyl isoquinoléine, en particulier oxyacanthine, cépharanthine ; une substance inhibitrice de PAF, en particulier un extrait de Gingko biloba, une substance inhibitrice d'enzymes PGE2.

12. Procédé non thérapeutique de soin cosmétique de la peau destinée aux peaux sensibles ou à combattre la sensibilité cutanée par stimulation de la production de bêta-endorphine par les kératinocytes de la peau, **caractérisé en ce qu'**il comprend l'application sur la partie de la peau concernée d'une composition cosmétique contenant un agent actif choisi dans le groupe du stachyose, du ciceritol et des extraits de plante contenant du stachyose ou du cicéritol.

13. Procédé selon la revendication 13, **caractérisé en ce que** ledit agent actif agit sur la production de la bêta-endorphine par les kératinocytes de la peau.

14. Procédé selon l'une des revendications 12 ou 13, **caractérisé en ce que** ledit agent actif est utilisé en tant qu'agent de soin des peaux sensibles.

15. Procédé selon l'une des revendications 12 à 14, **caractérisé en ce que** ledit agent actif est le stachyose.

16. Procédé selon l'une des revendications 12 à 14, **caractérisé en ce que** ledit agent actif est le ciceritol.

17. Procédé selon l'une des revendications 12 à 14, **caractérisé en ce que** ledit agent actif est contenu dans un extrait de plantes choisies dans le groupe constitué de la plante Tephrosia, du soja, du pois chiche, du lupin et de la lentille.

18. Procédé selon la revendication 17, **caractérisé en ce que** ledit extrait de plantes est un extrait de Tephrosia purpurea.

19. Procédé selon la revendication 17 ou 18, **caractérisé en ce que** ledit extrait est un extrait de graines de Tephrosia purpurea.

20. Procédé selon l'une des revendications 12 à 19, **caractérisé en ce que** ledit agent actif est contenu dans une composition cosmétique, à une concentration exprimée en poids sec comprise entre 0,0001 % et 10 % par rapport au poids total de ladite composition.

21. Procédé selon la revendication 20, **caractérisé en ce que** ladite concentration est comprise entre 0,01 % et 5 % en poids par rapport au poids total de ladite composition.

22. Procédé selon l'une des revendications 12 à 21, **caractérisé en ce que** ledit agent actif est combiné à une autre substance active cosmétiquement acceptable, de préférence choisie dans le groupe constitué d'un acide alphahydroxylé en particulier l'acide salicylique et ses dérivés, l'acide lactique, glycolique, malique ; une substance inhibitrice de l'enzyme PLA2, telle qu'un extrait de la plante Phéllodendron amurense, de la plante Azadirachta indica ; un agent tensioactif en particulier de la famille du laurylsulfate ; une substance alcaloïde de préférence une bisbenzyl isoquinoléine, en particulier oxyacanthine, cépharanthine ; une substance inhibitrice de PAF, en particulier un extrait de Gingko biloba, une substance inhibitrice d'enzymes PGE2.

## Claims

1. Non therapeutic use in a cosmetic composition of at least one active agent selected from the group consisting of stachyose, ciceritol and extracts of plants containing stachyose or ciceritol, as cosmetic agent for the care of sensitive skins, or for fighting against skin sensitiveness, by stimulation of the production of beta-endorphin in the skin.

2. Use according to claim 1, **characterized in that** said active agent acts on the production of beta-endorphin by the keratinocytes of the skin.

3. Use according to one of claims 1 or 2, **characterized in that** said active agent is used as an agent for the care of sensitive skins.

4. Use according to one of claims 1 to 3, **characterized in that** said active agent is stachyose.

5. Use according to one of claims 1 to 3, **characterized in that** said active agent is ciceritol.

6. Use according to one of claims 1 to 3, **characterized in that** said active agent is contained in a plant extract, the plant being selected from the group consisting of Tephrosia, soybean, chick pea, lupin and lentil.

7. Use according to claim 6, **characterized in that** said plant extract is an extract of Tephrosia purpurea.

8. Use according to one of claims 6 or 7, **characterized in that** said extract is an extract of seeds of Tephrosia purpurea.

9. Use according to one of claims 1 to 8, **characterized in that** said active agent is contained in a cosmetic composition, at a concentration expressed by dry weight comprised between 0.0001% and 10% based on the total weight of said composition.

10. Use according to claim 9, **characterized in that** said concentration is comprised between 0.01% and 5% by weight, based on the total weight of said composition.

11. Use according to one of claims 1 to 10, **characterized in that** said active agent is combined with another cosmetically acceptable active substance preferably selected from the group consisting of an alphahydroxy acid, particularly salicylic acid and its derivatives, lactic, glycolic, malic acid; an inhibitor of the enzyme PLA2, such as an extract of the plant Phellodendron amurense or the plant Azadirachta indica; a surfactant, particularly of the laurylsulfate family; an alkaloid substance, preferably a bisbenzylisoquinoline and particularly oxyacanthine or cepharanthine; a PAF inhibitor, particularly a Gingko biloba extract; and an inhibitor of PGE2 enzymes.

12. Non-therapeutic method of cosmetic care of the skin meant for sensitive skins or for fighting skin sensitiveness, by stimulating the production of beta-endorphin by the keratinocytes of the skin, **characterized in that** it comprises the application on the part of the skin, which is concerned, of a cosmetic composition containing an active agent selected from the group consisting of stachyose, ciceritol and plant extracts containing stachyose or ciceritol.

13. Method according to claim 12, **characterized in that** said active agent acts on the production of beta-endorphin by the keratinocytes of the skin.

14. Method according to one of claims 12 or 13, **characterized in that** said active agent is used as agent for the care of sensitive skins.

15. Method according to one of claims 12 to 14, **characterized in that** said active agent is stachyose.

16. Method according to one of claims 12 to 14, **characterized in that** said active agent is ciceritol.

17. Method according to one of claims 12 to 14, **characterized in that** said active agent is contained in a plant extract, the plant being selected from the group consisting of Tephrosia, Soybean, chick pea, lupin and lentil.

18. Method according to claim 17, **characterized in that** said plant extract is an extract of Tephrosia purpurea.

19. Method according to claim 17 or 18, **characterized in that** said extract is an extract of seeds of Tephrosia purpurea.

20. Method according to one of claims 17 to 19, **characterized in that** said active agent is contained in a cosmetic composition, at a concentration expressed by dry weight comprised between 0.0001% and 10% based on the total weight of said composition.

21. Method according to claim 20, **characterized in that** said concentration is comprised between 0.01% and 5% by weight, based on the total weight of said composition.

22. Method according to one of claims 12 to 21, **characterized in that** said active agent is combined with another cosmetically acceptable active substance preferably selected from the group consisting of an alphahydroxy acid, particularly salicylic acid and its derivatives, lactic, glycolic, malic acid; an inhibitor of the enzyme PLA2, such as an extract of the plant Phellodendron amurense or the plant Azadirachta indica; a surfactant, particularly of the laurylsulfate family; an alkaloid substance, preferably a bisbenzylisoquinoline and particularly oxyacanthine or cepharanthine; a PAF inhibitor, particularly a Gingko biloba extract; and an inhibitor of PGE2 enzymes.

## Patentansprüche

1. Nicht-therapeutische Verwendung von wenigstens einem aktiven Mittel, das gewählt ist aus der Gruppe aus Stachyose, Ciceritol und den Pflanzenextrakten, die Stachyose oder Ciceritol enthalten, als kosmetischem Mittel in einer kosmetischen Zusammensetzung für die Pflege empfindlicher Haut oder zur Bekämpfung der Hautempfindlichkeit durch Stimulierung der Produktion des Beta-Endorphins in der Haut.

2. Verwendung nach Anspruch 1, **dadurch gekennzeichnet, daß** das aktive Mittel durch die Keratinocyten der Haut auf die Produktion des Beta-Endorphins wirkt.

3. Verwendung nach einem der Ansprüche 1 oder 2, **dadurch gekennzeichnet, daß** das aktive Mittel als Pflegemittel empfindlicher Haut verwendet wird.

4. Verwendung nach einem der Ansprüche 1 bis 3, **dadurch gekennzeichnet, daß** das aktive Mittel Stachyose ist.

5. Verwendung nach einem der Ansprüche 1 bis 3, **dadurch gekennzeichnet, daß** das aktive Mittel Ciceritol ist.

6. Verwendung nach einem der Ansprüche 1 bis 3, **dadurch gekennzeichnet, daß** das aktive Mittel enthalten ist in einem Extrakt von Pflanzen, die gewählt sind aus der Gruppe, die aus der Pflanze Tephrosia, Soja, Kichererbsen, Lupine und Linse besteht.

7. Verwendung nach Anspruch 6, **dadurch gekennzeichnet, daß** der Pflanzenextrakt ein Extrakt von Tephrosia purpurea ist.

8. Verwendung nach einem der Ansprüche 6 oder 7, **dadurch gekennzeichnet, daß** der Extrakt ein Extrakt von Körnern von Tephrosia purpurea ist.

9. Verwendung nach einem der Ansprüche 1 bis 8, **dadurch gekennzeichnet, daß** das aktive Mittel in einer kosmetischen Zusammensetzung mit einer Konzentration enthalten ist, die, ausdrückt in Trockengewicht, zwischen 0,0001% und 10% im Verhältnis zum Gesamtgewicht der Zusammensetzung liegt.

10. Verwendung nach Anspruch 9, **dadurch gekennzeichnet dadurch**, daß das die Konzentration zwischen 0,01 Gewichtsprozent und 5 Gewichtsprozent im Verhältnis zum Gesamtgewicht der Zusammensetzung liegt.

11. Verwendung nach einem der Ansprüche 1 bis 10, **dadurch gekennzeichnet, daß** das aktive Mittel mit einer anderen kosmetisch verträglichen Wirksubstanz kombiniert wird, die vorzugsweise gewählt ist aus der Gruppe, die besteht aus einer Alpha-Hyroxylsäure, insbesondere Salicylsäure und ihren Derivaten, Milchsäure, Glykolysäure, Maleinsäure; einer Inhibitorsubstanz des Enzyms PLA2, wie einem Extrakt der Pflanze Phellondendron amurens, der Pflanze Azadirachta indicha; einem Tensid, insbesondere der Laurylsulfatfamilie; einer Alkaloidsubstanz, vorzugsweise ein Bisbenzyl-Isoquinolin, insbesondere Oxyacanthin, Cepharanthin; einer Inhibitorsubstanz von PAF, insbesondere ein Gingko biloba, einer Inhibitorsubstanz von PGE2-Enzymen.

12. Nicht-therapeutisches Verfahren zur kosmetischen Pflege der Haut, bestimmt für die empfindliche Haut oder zur Bekämpfung der Hautempfindlichkeit durch Stimulierung der Produktion des Beta-Endorphins durch die Keratinocyten der Haut, **dadurch gekennzeichnet, daß** es das Auftragen auf die betroffene Hautpartie einer kosmetischen Zusammensetzung umfaßt, die ein aktives Mittel enthält, gewählt aus der Gruppe aus Stachyose, Ciceritol und Pflanzenextrakten, die Stachyose oder Ciceritol enthalten.

13. Verfahren nach Anspruch 12, **dadurch gekennzeichnet, daß** das aktive Mittel durch die Keratinocyten der Haut auf die Produktion des Beta-Endorphins wirkt.

14. Verfahren nach einem der Ansprüche 12 oder 13, **dadurch gekennzeichnet, daß** das aktive Mittel verwendet wird als Pflegemittel empfindlicher Haut.

15. Verfahren nach einem der Ansprüche 12 bis 14, **dadurch gekennzeichnet, daß** das aktive Mittel Stachyose ist.

16. Verfahren nach einem der Ansprüche 12 bis 14, **dadurch gekennzeichnet, daß** das aktive Mittel Ciceritol ist.

17. Verfahren nach einem der Ansprüche 12 bis 14, **dadurch gekennzeichnet, daß** das aktive Mittel enthalten ist in einem Extrakt von Pflanzen, die gewählt sind aus der Gruppe, die aus der Pflanze Tephrosia, Soja, Kichererbsen, Lupine und Linse besteht.

18. Verfahren nach Anspruch 17, **dadurch gekennzeichnet, daß** der Pflanzenextrakt ein Extrakt von Tephrosia purpurea ist.

19. Verfahren nach Anspruch 17 oder 18, **dadurch gekennzeichnet, daß** der Extrakt ein Extrakt von Körnern von Tephrosia purpurea ist.

20. Verfahren nach einem der Ansprüche 12 bis 19, **dadurch gekennzeichnet, daß** das aktive Mittel in einer kosmetischen Zusammensetzung mit einer Konzentration enthalten ist, die, ausdrückt in Trockengewicht, zwischen 0,0001% und 10% im Verhältnis zum Gesamtgewicht der Zusammensetzung liegt.

21. Verfahren nach Anspruch 20, **dadurch gekennzeichnet dadurch**, daß die Konzentration zwischen 0,01 Gewichtsprozent und 5 Gewichtsprozent im Verhältnis zum Gesamtgewicht der Zusammensetzung liegt.

22. Verfahren nach einem der Ansprüche 12 bis 21, **dadurch gekennzeichnet, daß** das aktive Mittel mit einer anderen kosmetisch verträglichen Wirksubstanz kombiniert wird, die vorzugsweise gewählt ist aus der Gruppe, die besteht aus einer Alpha-Hyroxylsäure, insbesondere Salicylsäure und ihren Derivaten, Milchsäure, Glykolysäure, Maleinsäure; einer Inhibitorsubstanz des Enzyms PLA2, wie einem Extrakt der Pflanze Phellondendron amurens, der Pflanze Azadirachta indicha; einem Tensid, insbesondere der Laurylsulfatfamilie; einer Alkaloidsubstanz, vorzugsweise ein Bisbenzyl-Isoquinolin, insbesondere Oxyacanthin, Cepharanthin; einer Inhibitorsubstanz von PAF, insbesondere ein Gingko biloba, einer Inhibitorsubstanz von PGE2-Enzymen.
